# EUROPEAN PATENT SPECIFICATION

(11) **EP 0 787 495 B1**
(45) Date of publication and mention of the grant of the patent: **27.04.2005**
(21) Application number: 96101529.4
(22) Date of filing: 02.02.1996
(51) Int. Cl.: A61K 35/78, A61P 1/04

(54) **A process for the isolation of an active principle from Azadirachta indica useful for controlling gastric hyperacidity and gastric ulceration**
Verfahren zur Isolierung eines Wirkstoffes aus Azadirachta Indica zur Verwendung bei der Therapie von Hyperacidität und Magengeschwüren
Procédé d'isolation d'un principe actif d'Azadirachta indica utile dans le traitement de l'ulcération gastrique et l'hyperacidité

(43) Date of publication of application: 06.08.1997
(73) Proprietor: Council of Scientific & Industrial Research, New Delhi 110001 (IN)
(72) Inventor: Bandyopadhyay, Uday, Dr., c/o Indian Inst. of, Calcutta 700032 (IN); Chatterjee, Ratna, Dr., c/o Indian Inst. of, Calcutta 700032 (IN); Kumar Bandyopadhyay, Ranajit, Dr., c/o Indian Inst, Calcutta 700032 (IN)
(74) Representative: WILHELMS, KILIAN & PARTNER Patentanwälte

(56) References cited:
- EP-A- 0 617 119
- DATABASE WPI Week 8950 Derwent Publications Ltd., London, GB; AN 89-367647 XP002007272 & JP-A-01 275 602 (TERUMO CORP) , 6 November 1989
- PLANTA MEDICA, vol. 59, 1993, pages 215-217, XP000196198 G. P. GARG ET AL : "The Gastric Antiulcer Effects of the Leaves of the Neem Tree"
- THE JAPANESE JOURNAL OF PHARMACOLOGY , vol. 36, no. 4, December 1984, pages 527-533, XP000196179 S.A. HANIFA MOURSI ET AL: "Effect of Melia Azedarach Fruits on Gipsing-Restraint Stress-Induced Ulcers in Rats"
- PLANTA MEDICA , vol. 50, no. 2, April 1984, pages 143-146, XP000196181 N. R. PILLAI ET AL: "Effects of Nimbidin on Acute and Chronic Gastro-duodenal Ulcer Models in Experimental animals"
- PLANTA MEDICA, vol. 57, no. 1, February 1991, pages 65-68, XP000196182 J.M. VAN DER NAT ET AL: "Activity-Guided Isolation and Identification of Azadirachta indica Bark Extract Constituents which Specifically Inhibit Chemiluminescence Production by Activated Human Polymorphonuclear Leukocytes "

## Description

This invention relates to a process for the isolation of an active principle from *Azadirachta indica* useful for controlling gastric hyperacidity and gastric ulceration. Hyperacidity or hyperchlorhydria, a burning global problem, from which about 5% of world population is suffering today, is due to excess secretion of hydrochloric acid (HCl) from the gastric mucosa. The cause of this hypersecretion is obscure except that it is due to hyperactivity of the proton pumping H⁺-K⁺-ATPase of the parietal cell (Sachs, Penny & Lewin. Physiol. Rev. 58, 106, 1978). Hyperacidity may originate from stress and tension in our daily life. It may result from nonsteroidal and steroidal antiinflammatory drug therapy for treatment of some pathological conditions. Constant secretion of HCl also prevents mucosal wound healing and aggravates gastric ulceration, a disease caused by disbalance between some aggresive factors and cytoprotective factors of the gastric mucosa. Acid secretion is controlled by an interplay of physiological secretagogues. Histamine interacts with the H₂ receptor of the parietal cell and triggers acid secretion through elevation of cAMP and Ca⁺⁺ leading to the activation of H⁺-K⁺-ATPase (Sachs, Carlsson, Londberg & Wallmark, Ann. Rev. Pharmacol. Toxicol. 28, 269, 1988). Acetylcholine interacts with the muscarinic receptor and stimulates HCl secretion by increasing intracellular diacylglycerol, IP₃ and Ca⁺⁺ (Muallem & Sachs, Amer. J. Physiol. 248, C216, 1985). Gastrin does this by transient hike of intracellular Ca⁺⁺ by interacting with the gastric receptor (Soll, Amerian, Thomas, Ready & Elashoft, J. Clin. Inv., 73, 1434, 1984). How cAMP, Ca⁺⁺ or IP₃, the second messengers, transmits the signal to the H⁺-K⁺-ATPase is still obscure.

The medications to combat hyperacidity and ulceration (Breenton. In Goodman's & Gilman's The Pharmacological Basis of Therapeutics, eds. Gilman, Rall, Nies & Taylor, 2, 897, 1991) are of three types : [1] H₂-receptor blockers which block the occupancy of the secretagogue with the receptor causing uncoupling of stimulus-secretion coupling, e.g., cimetidine, ranitidine, famotidine and nizatidine; muscarinic cholinergic receptor blockers such as atropine, pirenzepine and telenzepine; [ii] Inactivator of H⁺-K⁺-ATPase, e.g. omeprazole; [iii] Different antacids (alkali gels, e.g., Al(OH)₃, Mg(OH)₂ etc.).

A variety of adverse reactions have been reported for these medications (Breenton. In Goodman's & Gilman's The Pharmacological Basis of Therapeutics. eds. Gilman, Rall, Nies & Taylor, 2, 897, 1991). Cimetidine causes altered laxation, headache, dizziness and nausea, myalgia, skin rashes and itching, incidence of symptoms related to the central nervous system (CNS), impaired renal function, loss of libido, impotence and gynecomastia (Howden & Hunt. Peptic ulcer disease. In A Pharmacological Approach to Gastrointestinal Disorders. ed. Lewis J. 3, 1994). Cimetidine also inhibits cytochrome P₄₅₀-catalyzed drug oxidation and hydroxylation of estradiol to increase the plasma concentration in men. Cimetidine occasionally causes hematological effects (various cytopenias) and alters function of immune system. Ranitidine also has several side effects (Howden & Hunt, Peptic ulcer disease, in A Pharmacological Approach to Gastrointestinal Disorders, ed. Lewis J. 3, 1994) and causes headache, mild diarrhoea and a reversible form of drug-induced hepatitis in some patients. It also causes reversible mental confusion in some sick, elderly patients. Very little is known about famotidine and nizatidine as they have been marketed recently. Omeprazole causes gastrointestinal troubles including nausea, diarrhoea and abdominal colic and CNS effects (e.g. headache, dizziness, somnolence). Skin rash, leucopenia and transient elevation of plasma activities of hepatic aminotransferases have been observed occasionally. Omeprazole reduces the secretion, synthesis and gene expression of pepsinogen in rat stomach (Kakel, Ichinore, Tsukada, Tatematsu, Tezuka, Yahagi, Matsushima, Miki, Kurokawa, Takahashi & Fukamachi. Biochem. Biophys. Res. Commun. 195, 997, 1993). Gastric mucosal hypertrophy and carcinoma have been reported with long term therapy of omeprazole (Ivy, Amer. J. Med. 84, Suppl.2A, 41, 1988). Antacids such as Al(OH)₃ causes constipation while Mg(OH)₂ causes loose stools or diarrhoea. In person with impaired renal function long term administration of Al³⁺ even initiates osteodystrophy, proximal myopathy and encephalopathy; the latter may take the form of dementia or seizures. Considering the side effects and disadvantages of these drugs, identification of a better drug having less toxicity but potent acid and ulcer inhibitory activity is urgently required to control these human sufferings. Natural product having therapeutic value is accepted as less toxic for human medication and attempt to identify such product is always welcome.

Plants are part and parcel of human society from the dawn of civilization and extensive uses of neem (Margosa tree) for the treatment of a variety of human diseases are reminiscent of broad spectrum activities of some modern medicines. *Azadirachta indica* A. Juss (syn. Melia azadirachta) and *Melia azedarach* Linn are the two closely related species of the Meliaceae. The former is popularly known as the Indian neem or the Indian lilac and the latter as the Persian lilac. *A. indica* is an evergreen tree cultivated in various parts of India and almost every part of this plant has been used in the folk medicine in India from antiquity (Chopra, Chopra, Handa & Kapur. Indigenous drugs of India. Dhur & Sons Pvt. Ltd., Calcutta, 360, 1958). The aqueous extract of bark is regarded as tonic, astringent and useful in fever, vomiting and skin diseases; the leaves are beneficial in all types of anorexia and in skin diseases and the fruits are described as purgative and emolient and are useful in the treatment of intestinal worms, urinary diseases and piles (Chatterjee & Pakrashi. The treatise on Indian Medicinal Plants. eds. Chatterjee & Pakrashi, 3, 76, 1994). The water soluble fraction of the alcoholic extract of the green leaves has been found to possess significant hypoglycemic property (Murty, Rao, Rao & Murty. Ind. J. Pharmacol. 10, 247, 1978; Pillai & Shantakumari. Ind. J. Med. Res. 74, 931, 1981). The aqueous extract of neem leaves possesses gastric antiulcer activity (Garg, Nigam & Ogle. Planta Med. 59, 215, 1993).

The juice of the leaves of *M. azadarach* is said to be anthelmentic and emmenagogue. The root is useful in the treatment of tumors (Japanese Patent. Terumo Corporation, 2,522,001, 1983, C.A. 10000 168227), pain in the heart, leucoderma and blood impurities. The leaves and bark are used internally and externally in leprosy and scrofula, in the treatment of eczema and relief of asthmatic attacks.

Azadirachtin, an oxygenated complex tetranotriterpenoid isolated from neem fruit ethanol extract, is widely used as a pesticide having phagorepellent, antifeadent and systemic growth disruptor properties in insects (Butterworth, Morgan & Percy. J. Chem. Soc. Perkin. Trans. 1, 2445, 1972; Gill & Lewis, Nature, 236, 159, 1972; Zanno, Miura, Nakanishi & Editor. J. Amer. Chem. Soc. 97, 1975, 1975; Aldhons, Science, 258, 893, 1992; Govindachari, Current Sci. 63, 117, 1992; Grossman & Ley, Tetrahedron, 50, 11553, 1994). Azadirachtin has recently been shown to be active against larva of *P. xylostella* L (Verkerk & Wright, Pest. Sci. 37, 83, 1993) and inhibitory to the development of malarial parasites (Jones, Delholm, Ley, Lovell, Wood & Sinden. FEM Microb. Letts. 120, 267, 1994). Neem oil also possesses anti-fertility property (Juneja & Williams. Pharmacol. Letts. Life Sc. 53, 279, 1993; Upadhyay, Dhawan, Sharma & Talwar. Contraception, 49, 161, 1994; Kaushic & Upadhyay, Contraception, 51, 203, 1995). Spermicidal, diuretic and anti-inflammatory effects of neem are also reported (Sharma & Saxena. Ind. J. Med. Res. 13, 1038, 1959; Shah, Sheth., Vide & Shah. Ind. J. Med. Res. 12, 150, 1958; Pillai & Santakumari. Planta Med. 43, 59, 1981). Nimbidin, the bitter principle of neem, has been shown to have antigastric ulcer activity (Pillai, Suganthan, Seshadri & Santakumari. Ind. J. Med. Res. 68, 169, 1978). In fact neem has become cynosure of world wide research effort today for its beneficial use in present day civilization. More than 100 different compounds have been isolated and characterized from neem (Chatterjee & Pakrashi. The treatise on Indian Medicinal Plants, 3, 76, 1994).

The main object of the present invention is to provide a process for the isolation of an active principle from the plant *Azadirachta indica* which is useful for controlling gastric hyperacidity and gastric ulceration. Accordingly, the present invention provides a process for the isolation of an active principle which is a phenolic glycoside from *Azadiracha indica* (neem) useful in the treatment of gastric hyperacidity and gastric ulceration according to claim 1.

Preferred embodiments of the inventive process are covered by claims 2 to 6.

The HPLC column used is a reverse-phase C₁₈ column like u Bondapak, Novapak or Deltapak. The residue in step [d) is subjected to HPLC at a flow rate ranging from 0.5 ml/min - 3 ml/min for collecting the eluate in the period in the range of 6 min - 60 min. The phenolic glycoside coming out of the column are detected from their maximum absorbance at 280 nm which shifts to 293 nm by addition of alkali and gives positive Molisch's test for carbohydrate.

Different parts of the *Azadirachta indica* (neem) such as leaves, flowers, bark are soaked in water in a conical flask. The bark of plant is preferred. The flask is shaken occassionally. The pH of the solution may be maintained between 5.5-7.0 by the addition of HCl or bicarbonate whichever is required. The water extract is filtered and then lyophilized by freeze drying. The lyophilised powder is first extracted with solvents with increasing polarity. The solvent systems which can be used for the purpose may be any one of the followig :
(i) petroleum ether, ethyl acetate, methanol and butanol; (ii) petroleum ether, butanol, ethanol, and acetone; (iii) petroleum ether, chloroform, butanol, and methanol: (iv) petroleum ether, dichloroethane, 2-propanol and methanol.

The residue obtained after extracting with the highest polar solvent is subjected to HPLC using reverse-phase column capable of separating molecules based on hydrophobicity. The fractions from the column containing the phenolic glycoside having maximum absorption at 280 nm is taken and lyophilized to **ISOLATION OF THE ACTIVE PRINCIPLE :**

### Extraction and preparation of test samples :

The following examples are given by way of illustration of the process of the present invention and should not be construed to limit the scope of the present invention.

### Example 1 :

100 gm of bark of *Azadirachta indica* in small fine pieces and air dried in the shade was soaked in 1,000 ml of glass distilled water for 30 hr in a three litre conical flask at room temperature (20-30^{o}C). The flask was shaken occasionally. The water extract, brown red in colour, was filtered through Whatman No. 1 and then lyophilised. The lyophilised powder (3 gm) was first extracted with 100 ml petroleum ether (b.p. 60-80°C). The petroleum ether extract was discarded. The residue was subsequently extracted with 100 ml ethylacetate. The ethylacetate insoluble part was then extracted with 150 ml methanol and finally methanol insoluble part was extracted with 100 ml n-butanol. Methanol soluble part showed some activity. n-Butanol insoluble part showed acid inhibitory and antiulcer activity in both *in vivo* and *in vitro* experiments. The n-butanol insoluble part was further resolved in HPLC using preparative C₁₈ deltapak column and eluted with 50 : 50 = methanol : water at a flow rate of 1.5 ml/min. When scanned at 280 nm, n-butanol insoluble part (which is soluble in water or in 50 : 50 water : methanol) was resolved into six peaks with retention time of 24 min, 34 min, 35 min, 35.5 min, 38.5 min, and 40 min and were designated as peak 1, peak 2, peak 3, peak 4, peak 5 and peak 6 respectively. Peak 1 showed the acid inhibitory activity. Homogeneity of peak 1 was tested in refractive index detector and also in 2D-TLC (solvent, ethylacetate : methylethylketone : formic acid : water = 4 : 3 : 1 : 2 or in methanol : water - 95 : 5). Homogeneity of peak 1 was further confirmed using C₁₈ ion-pair column equipped with electrochemical detector. Peak 1 was lyophilised to obtain a brown powder which is a phenolic glycoside and stored at 4^{o}C. The yield of the active principle is 7%.

### Example 2

90 gm of the airdried bark in small pieces was soaked in 1,500 ml of glass distilled water (pH = 6.2-6.5) for 35 hr in a three litre conical flask at room temperature (25-35^{o}C). The flask was continuously shaken in a shaker bath. The water extract, brown in colour, was filtered through Whatman No. 1 filter paper and then lyophilised. The lyophilised powder (3.5 gm) was first extracted with 150 ml petroleum ether (b.p. 60-80^{o}C). The petroleum ether extract was discarded. The residue was subsequently extracted with 100 ml ethylacetate. The ethylacetate insoluble part was then extracted with 150 ml ethanol and finally ethanol insoluble part was extracted with 100 ml propanol. Ethanol soluble part showed some biological activity. Propanol insoluble part showed acid inhibitory, antisecretory and antiulcer activity in both *in vivo* and *in vitro* experiments. The propanol insoluble part was further resolved in HPLC using preparative C₁₈ deltapak column and eluted with 50 : 50 = ethanol : water at a flow rate of 1.5 ml/min. When scanned at 280 nm, propanol insoluble part was resolved into six peaks with retention time of 24 min, 34 min, 35 min, 35.5 min, 38.5 min and 40 min and were designated as peak 1, peak 2, peak 3, peak 4, peak 5, and peak 6 respectively. Peak 1 showed the acid inhibitory activity. Homogeneity of peak 1 was tested in refractive index detector and also in 2D-TLC (solvent, ethylacetate : methylethylketone : formic acid : water = 4:3:1:2, or in methanol : water = 95 : 5). Homogeneity of peak 1 was further confirmed using C₁₈ ion-pair column equipped with electrochemical detector. Peak 1 was stored (as lyophilised powder) after removal of ethanol and water in a rotavapour to obtain a powder which is n phenolic glycoside. Yield of this active principle is 5%.

### Example 3

100 gm of air dried leaves were crushed and soaked in 1,000 ml of glass distilled water (pH 6.2-6.5) for 48 hrs in a three litre conical flask at room temperature (25°C). The flask was continuously shaken and the aqueous extract was filtered through glasswool and then lyophilised. This extract is biologically active in inhibiting acid secretion and gastric ulceration. The lyophilised powder (1 gm) was first extracted with 50 ml petroleum ether (b.p. 60-80^{o}C) and the petroleum ether extract was discarded. The residue was subsequently extracted with 50 ml of ethylacetate. The ethylacetate insoluble part was then extracted with 100 ml methanol and finally methanol insoluble part was extracted with 50 ml n-butanol. n-Butanol insoluble part showed acid inhibitory, antisecretory and antiulcer activity in both *in vivo* and *in vitro* experiments. n-Butanol insoluble part was further resolved in HPLC using a preparative C₁₈ column and eluted with 60 : 40 = methanol : water at a flow rate of 1.0 ml/min. When scanned at 280 nm, n-butanol insoluble material resolved into four peaks with retention time of 36 min, 46 min, 47 min and 48 min and were designated as peak 1, peak 2, peak 3 and peak 4 respectively. Peak 1 showed acid inhibitory activity. Homogeneity of peak 1 was tested as done in Examples 1 and 2. Peak 1 was lyophilised to obtain a brown powder which is a phenolic glycoside. The yield of the active principle is 2%.

The active principle isolated according to the process of the present invention has been identified as a phenolic glycoside and has the following properties :

### Physical :

1. The lyophilised powder is brick-red in colour, readily soluble in water at a concentration upto 60 mg/ml and in DMSO at 50 mg/ml.
2. In UV spectrometry, the aqueous solution gives symmetrical peak at 224 nm and 276 nm. When a drop of concentrated KOH is added, only 276 nm peak is shifted to 298 nm and the original brick red colour turned into deep red. The original colour reappears on addition of acetic acid. This character is shown by standard phenolic compound.
3. In IR spectroscopy (in KBr), this material gives peak at 3380 cm⁻¹, 2362 cm⁻¹, 2360 cm⁻¹, 1514 cm⁻¹, 1512 cm⁻¹, 1452 cm⁻¹, 1452 cm⁻¹, 1382 cm⁻¹ and 1067 cm⁻¹. Some of the absorptions are due to hydroxyl group.
4. Melting point is above 260°C.
5. Optical rotation of this material is, [ alpha ]²⁵ + 14° (C 1.0, H₂O).
6. ¹H NMR of this material indicates peaks at 8.01, 7.87, 5.93, 4.69, 4.59, 4.50, 4.30, 3.94, 3.88, 3.69, 2.30, 2.19, 2.14, 2.01, 1.91, 1.80, 1.55, 1.16, 1.12, 0.8, 0.32, 0.26 and 0.15 PPM in D₂O in 200 MHz/52 MM NMR spectrometer.
7. ¹H NMR of the isolated aglycan part of this material in DMSO using 200 MHz/52 MM NMR spectrometer shows peaks at 8.28, 3.42, 3.39, 3.15, 2.81 and 2.49 PPM.

### Chemical :

1. When elemental analysis (particularly C,H,N) was carried out in CHN analyser, it gives 44.76% carbon and 4.72% hydrogen but no nitrogen.
2. It gives blue colouration with Folin Ciocalteau reagent which is positive for phenolic group. No peptide was detected in polyacrylamide gel electrophoresis.
3. Molisch's test for carbohydrate is positive. When hydrolised, the glycan part consists of arabinose, glucose, mannose, rhamnose and galactose in the ratio of 1:1:1:1:2 as detected by automated gas-liquid chromatography. By controlled digestion overnight at 28±1°C with 5% H₂SO₄, an aglycan part is precipitated which is insoluble in water but readily soluble in methanol. This aglycan part is phenolic in nature as revealed by (1) its UV absorption at 279 nm which shifts to 293 nm by dilute alkali, and (2) its reduction of ferric ferricyanide to form a prussian blue colour of ferric ferrocyanide - a positive test for phenolic compound.
4. Magnetometer data indicates that it lacks any transition metal ion. Other metal ions, e.g. Na⁺, K⁺, Ca⁺⁺ etc. are also absent as evidenced from negative flame test. Further studies to determine the exact structure of this novel compound is in progress.

The compound obtained by the process of the present invention was subjected to bioassay studies :

### Animal :

Albino rats (wistar strain of either sex, 180-200 gm) were deprived of food but allowed free access to water 24 hr before the start of experiment and kept in cages with meshed aluminium base to avoid caprophagy.

### In vivo studies :

### A. Pylorus ligation-induced gastric acid secretion :

Pyloric ligation was done under light ether anaesthesia (Shay, Komarov, Fels, Meranze, Gruenstein & Siplet. Gastroenterology, 5, 43, 1945) 30 min after intraperitoneal injection of different doses of active principle (0.5-2.5 mg/100 gm). They were sacrificed by decapitation 2 hr after ligation. The gastric content was collected by flushing the stomach cavity with 2 ml of 0.9% saline through the pyloric end (Bandyopadhyay, Bhattacharyya, Chatterjee & Banerjee, Biochem. J. 284, 305, 1992). It was centrifuged at 5,000g for 10 min in a RC-5B refrigerated Sorvall centrifuge. The clear supernatant was collected, the volume recorded and HCl content was measured by autotitration in a pH meter, Radiometer, Copenhagen.

### B. Mercaptomethylimidazole (MMI)-induced gastric acid secretion :

MMI-induced gastric acid seretion was measured as described previously (Bandyopadhyay, Bhattacharya, Chatterjee & Banerjee. Biochem. J. 284, 305, 1992). Rats fasted for 24 hr were injected with active principle at different doses (0.5-2.5 mg/100 gm) and then after 30 min MMI (3 mg/200 gm) or vehicle (water) was injected (IP). After 2.5 hr, the animals were killed, abdomen was opened and the gastric fluid was collected as described. This active principle dose-dependently blocks MMI-induced gastric acid secretion.

### In vitro studies :

### A. Measurement of accumulation of [¹⁴C]-aminopyrine [¹⁴CAP] in isolated gastric glands as an index of acid secretion :

Gastric glands were prepared by controlled enzymatic digestion (Berglindh & Obrink. Acta Physiol. 96, 150, 1976). Briefly, rabbit gastric mucosa was scraped and chopped finely with scissors. Digestion was performed for 25 min at 37°C using 20 mg collagenase/50 ml incubaton medium (NaCl, 140 mM; MgSO₄ 7H₂O 1.2 mM; CaCl₂ 2H₂O 1 mM; Hepes, 10 mM; KOH, 5.4 mM; Cimetidine, 100 uM; D(+) glucose, 0.5 mg/ml; BSA, 2 mg/ml). The glands were washed three times with incubation medium and resuspended in the same medium. [¹⁴C]-AP was added to the gland suspension in presence and absence of different secretagogue and 1 ml aliquots were removed at 0, 15, 30 and 60 min. The samples were centrifuged briefly, the supernatant was removed, the pellet was dried and dissolved in 3N KOH. Radioactivity was determined by liquid scintillation counting. AP accumulation is expressed as CPM/mg of gland (Berglindh. Acta Physiol. Scand. 96, 150, 1976).

### B. Assay of H⁺-K⁺-ATPase activity :

**Preparation of gastric vesicles enriched in H**^{**+**}**-K**^{**+**}**-ATPase :** Hog gastric mucosal microsome was prepared as described (Soumarmon, Abasfado, Bonfils & Lewine. J. Biol. Chem. 255, 11682, 1980; Wolosin & Forte. J. Biol. Chem. 256, 3149, 1981). Briefly, the scrapped fundic mucosa was washed with physiological saline and homogenized in a buffer containing 250 mM sucrose, 2 mM MgCl₂, 1 mM EGTA and 2 mM Tris-Cl (pH 7.4). The post-mitochondrial supernatant was spun at 100,000g for 60 min to get the microsomal pellet. This was suspended in homogenizing buffer and layered over a discontinuous sucrose gradient composed of equal volume (10 ml) of 37% and 22% (w/v) sucrose solution (sucrose solutions were made with buffer containing 1 mM EGTA and 2 mM Tris-Cl). Following centrifugation for 12 hr at 81,000g in a Beckman ultracentrifuge, the membrane band at the interface of 22% and 37% sucrose layers was collected and used as the (H⁺-K⁺)ATPase enriched vesicular fraction. This fraction was stored in buffer at 1 mg/ml and kept at -20°C for several months. All steps were carried out at 4^{o}C.

(H⁺-K⁺)ATPase activity was determined as described (Beil, Hackbarth & Sewing. Brit. J. Pharmacol. 88, 19, 1986). Briefly, the enzyme activity was measured in 1 ml of an incubation medium containing 2 mM MgCl₂, 50 mM Tris-HCl buffer (pH 7.4), 0.1 mM EGTA, 5-10 ug membrane protein with or without 7 mM KCl and 7 mM NH₄Cl. It was preincubated for 2 min at 37^{o}C, the reaction was started by 20 ul of 0.1 M ATP (final concentration 2 mM) and incubated for 10 min at 37°C. After stopping the reaction by 0.1 ml of 50% cold TCA, inorganic phosphate liberated was measured as described (Staussky & Shorr. J. Biol. Chem. 202, 675, 1953). (H⁺-K⁺)ATPase activity was calculated from the difference in the amount of phosphate released in the medium in presence or absence of 7 mM KCl and 7 mM NH₄Cl. Effect of the active principle was studied at varying concentration before the addition of ATP.

This compound, isolated according to the process described in Example 1, inhibits both pylorus-ligated and mercaptomethyl-imidazole (MMI)-induced gastric acid and volume secretion *in vivo* dose-dependently. At a dose of 14 ug/100 gm rat, it completely blocks acid secretion. It also reduces 70% and 62% of the volume secretion in pylorus-ligated and MMI-injected animals respectively.

In *in vitro* gastric gland preparation, it inhibits completely both basal and secretagogue-induced acid secretion at a concentration of 3 mg/ml gland suspension. The compound isolated according to process of the present invention inhibits (H⁺-K⁺)ATPase activity in a concentration dependent manner. At a concentration of 2 ug/ml, it completely inhibits the (H⁺-K⁺)ATPase. When compared, this compound isolated according to the proces of the present invention, is at least three times more potent than omeprazole.

The active principle obtained by the process described in Example 2, behaved similarly as the compound isolated according to the process described in Example 1, in inhibiting *in vivo* and *in vitro* gastric acid secretion as well as gastric ulceration. At a dose of 20 ug/100 gm rat, it blocked acid secretion completely and reduced 60% and 65% of the volume of gastric secretion in pylorus-ligated and MMI-injected animals respectively. In *in vitro* gastric gland preparation, it inhibited both basal and secretagogue-induced acid secretion at a concentration of 4 mg/ml gland suspension. It also completely inhibited (H⁺-K⁺)ATPase activity at a concentration of 3 ug/ml.

### Advantages of the present invention :

1. The phenolic glycoside isolated is useful for controlling gastric hyperacidity and gastric ulceration. This novel compound is effective at a very low concentration. No death was observed when rats were exposed to a high dose (2.0 mg/100 g rat). Thus, 14 ug/100 g rat, a dose which completely inhibits acid secretion is neither lethal nor toxic.
2. IC₅₀ value of the novel compound is lower than omeprazole. The compound is more potent than omeprazole for complete inhibition of H⁺-K⁺-ATPase.
3. The yield of the compound (from the lyophilised powder) is very high (7-8%).
4. As the novel compound isolated does not affect significantly pepsinogen secretion in gastric fluid, it may be selectively used as a drug for inhibiting hyperacidity. As acid delays ulcer healing, it is beneficial in healing gastric ulcer. It also blocks stress ulcer (Das & Banerjee. Mol. Cell. Biochem. 125, 115, 1993) by 90% at a concentration of 2 mg/100 g rat.
5. The isolated novel compound is obtained by extraction with water and its phenolic glycoside character is entirely different from the compounds isolated so far from neem tree as reported earlier (Mahato, Sahu & Poddar. Science & Culture 53, Suppl.No. 5, 1987; Chatterjee & Pakrashi. The treatise on the Indian Medicinal Plants. eds. Chatterjee & Pakrashi, 3, 76, 1994).

## Claims

1. A process for the isolation of an active principle from *Azadirachta indica* (neem) useful in the treatment of gastric hyperacidity and gastric ulceration which comprises
[a] preparing an aqueous extract from parts of *Azadirachta indica* (neem) by soaking small pieces of the neem plants with destilled water of P_{H} 5,5 - 7,0 over 3 - 48 hrs at ambient temperature and separating undissolved impurities by known methods ;
[b] lyophilising the said extract obtained in step [a] by conventional methods to form powder ;
[c] extracting the lyophilised powder with solvents of increasing polarity selected from petroleum ether, ethylacetate, methanol, butanol; petroleum ether, butanol, ethanol, acetone; petroleum ether, chloroform, butanol, methanol; petroleum ether, dichloroethane ;
[d] subjecting the residue obtained in step [c] to HPLC on a reverse-phase column capable of separating molecules having different hydrophobicities ;
[e] separating the fraction coming out of the column which has a maximum absorption of UV at 280 nm ;
[f] lyophilizing the eluate obtained in setp [d] by known methods to form powder of the active principle.

2. A process as claimed in claim 1 wherein the parts of the plants used are the leaves flowers and bark, the bark being the preferred one.

3. A process as claimed in claims 1-2 wherein the lyophilization of the extract to form a powder is effected by freeze drying.

4. A process as claimed in claims 1-3 wherein the solvents with increased polarity used for extracting the lyophilized powder is selected from:
i) petroleum ether, ethylacetate, methanol, butanol;
ii) petroleum ether, butanol, ethanol, acetone;
iii) petroleum ether, chloroform, butanol, methanol;
iv) petroleum ether, dichloroethane, 2-propanol, methanol

5. A process as claimed in claims 1-4 wherein the HPLC column used is a reverse-phase C₁₈ column.

6. A process as claimed in claims 1-5 wherein the residue in step [d] is subjected to HPLC at a flow reate ranging from 0.5 ml/min. -3.0 ml/min and the eluate coming out of the column during the period in the range of 6 min- 60 min containing the phenolic glycoside is collected.

## Patentansprüche

1. Verfahren zur Isolierung eines Wirkstoffs aus *Azadirachta indica* (Neem) zur Verwendung bei der Behandlung von Übersäuerung des Magens und Magengeschwüren mit den Stufen:
[a] Herstellen eines wässrigen Extrakts aus Teilen von *Azadirachta indica* (Neem) durch Weichen kleiner Teile der Neem-Pflanzen mit destilliertem Wasser mit einem pH-Wert von 5,5 - 7,0 über einen Zeitraum vom 3 - 48 Stunden bei Raumtemperatur und Abtrennen nicht gelöster Verunreinigen durch bekannte Verfahren,
[b] Lyophilisieren des in Stufe [a] erhaltenen Extrakts durch konventionelle Verfahren zur Bildung eines Pulvers,
[c] Extrahieren des lyophilisierten Pulvers mit Lösungsmitteln ansteigender Polarität, ausgewählt aus Petrolether, Ethylacetat, Methanol, Butanol; Petrolether, Butanol, Ethanol, Aceton; Petrolether, Chloroform, Butanol, Methanol; Petrolether, Dichlorethan,
[d] Behandeln des in Stufe [c] erhaltenen Rückstands in einer HPLC auf einer Umkehrphasensäule, welche Moleküle verschiedener Hydrophobien trennen kann,
[e] Abtrennen der aus der Säule austretenden Fraktion, welche eine maximale UV-Absorption bei 280 nm aufweist,
[f] Lyophilisieren des in Stufe [d] erhaltenen Eluats durch bekannte Verfahren zur Bildung eines Pulvers des Wirkstoffs.

2. Verfahren gemäß Anspruch 1, wobei es sich bei den Teilen der verwendeten Pflanzen um Blätter, Blüten und Borke handelt, wobei die Borke bevorzugt ist.

3. Verfahren gemäß Anspruch 1 oder 2, wobei die Lyophilisation des Extrakts zur Bildung eines Pulvers durch Gefriertrocknen bewirkt wird.

4. Verfahren gemäß der Ansprüche 1 bis 3, wobei die Lösungsmittel mit ansteigender Polarität, die zum Extrahieren des lyophiliserten Pulvers verwendet werden, aus folgenden ausgewählt werden:
i) Petrolether, Ethylacetat, Methanol, Butanol,
ii) Petrolether, Butanol, Ethanol, Aceton,
iii) Petrolether, Chloroform, Butanol, Methanol,
iv) Petrolether, Dichlorethan, 2-Propanol, Methanol.

5. Verfahren gemäß der Ansprüche 1 - 4, wobei es sich bei der verwendeten HPLC-Säule um eine C₁₈-Umkehrphasensäule handelt.

6. Verfahren gemäß der Ansprüche 1 - 5, wobei der in Stufe [d] erhaltene Rückstand HPLC mit einer Fließgeschwindigkeit von 0,5 ml/Min. - 3,0 ml/Min. unterworfen und das Phenolglycosid enthaltende Eluat, welches in dem Zeitraum von 6 Min. - 60 Min. aus der Säule austritt, gesammelt wird.

## Revendications

1. Processus d'isolation d'un principe actif d'*Azadirachta indica* (neem) utile dans le traitement de l'hyperacidité gastrique et de l'ulcération gastrique, qui comprend les étapes consistant à :
[a] préparer un extrait aqueux à partir de parties d'*Azadirachta indica* (neem) par immersion de petites pièces des neems dans de l'eau distillée de pH 5,5 à 7,0 pendant 3 à 48 heures à température ambiante et par séparation des impuretés non dissoutes par des procédés connus ;
[b] lyophiliser ledit extrait obtenu dans l'étape [a] par des procédés conventionnels pour former une poudre ;
[c] extraire la poudre lyophilisée avec des solvants de polarité croissante choisis parmi l'éther de pétrole, l'acétate d'éthyle, le méthanol, le butanol ; l'éther de pétrole, le butanol, l'éthanol, l'acétone ; l'éther de pétrole, le chloroforme, le butanol, le méthanol ; l'éther de pétrole, le dichloroéthane ;
[d] soumettre le résidu obtenu dans l'étape [c] à une HPLC sur une colonne en phase inverse capable de séparer les molécules ayant des hydrophobicités différentes ;
[e] séparer la fraction sortant de la colonne qui a une absorption maximale des UV à 280 nm ;
[f] lyophiliser l'éluât obtenu à l'étape [d] par des procédés connus pour former une poudre du principe actif.

2. Processus selon la revendication 1, dans lequel les parties des plantes utilisées sont les feuilles, les fleurs et l'écorce, l'écorce étant la partie préférée.

3. Processus selon les revendications 1 et 2, dans lequel la lyophilisation de l'extrait pour former une poudre est réalisée par séchage par congélation (« freeze-drying »).

4. Processus selon les revendications 1 à 3, dans lequel les solvants ayant une polarité croissante utilisés pour l'extraction de la poudre lyophilisée sont choisis parmi :
i) l'éther de pétrole, l'acétate d'éthyle, le méthanol, le butanol ;
ii) l'éther de pétrole, le butanol, l'éthanol, l'acétone ;
iii) l'éther de pétrole, le chloroforme, le butanol, le méthanol ;
iv) l'éther de pétrole, le dichloroéthane, le 2-propanol, le méthanol.

5. Processus selon les revendications 1 à 4, dans lequel la colonne de HPLC utilisée est une colonne C₁₈ à phase inverse.

6. Processus selon les revendications 1 à 5, dans lequel on soumet le résidu dans l'étape [d] à une HPLC à un débit dans la plage de 0,5 mL/minute à 3,0 mL/minute et dans lequel on collecte l'éluât sortant de la colonne durant la période se trouvant dans la plage de 6 minutes à 60 minutes et contenant le glycoside phénolique.
